# EUROPEAN PATENT SPECIFICATION

(11) **EP 3 714 902 B1**
(45) Date of publication and mention of the grant of the patent: **28.08.2024**
(21) Application number: 20163014.2
(22) Date of filing: 13.03.2020
(51) Int. Cl.: A61K 47/26, A61K 9/00, A61K 9/08, A61K 47/18, A61K 31/00, A61K 39/00

(54) **STABILIZATION OF PHARMACEUTICAL COMPOSITIONS COMPRISING POLYSORBATE**
STABILISIERUNG VON PHARMAZEUTISCHEN ZUSAMMENSETZUNGEN MIT POLYSORBAT
STABILISATION DE COMPOSITIONS PHARMACEUTIQUES COMPRENANT UN POLYSORBATE

(43) Date of publication of application: 30.09.2020
(73) Proprietor: LEK Pharmaceuticals d.d., 1526 Ljubljana (SI)
(72) Inventor: Sink, Roman, 1526 Ljubljana (SI); Brovc, Ema Valentina, 1526 Ljubljana (SI); Pajk, Stane, 1526 Ljubljana (SI); Mravljak, Janez, 1526 Ljubljana (SI)
(74) Representative: Greiner, Elisabeth

(56) References cited:
- EP-A1- 3 156 071
- EP-A1- 3 607 969
- WO-A1-2018/060210
- WO-A1-2019/106206

## Description

### Field of the Invention

The present invention relates to stabilized pharmaceutical compositions comprising an improved histidine-based buffer system that reduces the degradation of polysorbates.

### Background of the Invention

Pharmaceutical compositions, in particular biopharmaceutical compositions, typically comprise an active agent, a surfactant and a buffer system, often complemented by chelating agents, stabilizers, tonicity modifiers and antioxidants. Excipients with well-established safety profiles are preferentially used due to the lower risks and costs associated with their approval by health authorities.

The most common surfactants used in the pharmaceutical industry are polysorbates and they are present in about 80% of commercial pharmaceutical compositions, in particular monoclonal antibody (mAb) compositions. Numerous different polysorbates are known and routinely added into formulations of these drugs to maintain adequate physical and chemical stability during storage, even at ambient temperatures. Polysorbates are amphiphilic, non-ionic surfactants. Among the known polysorbates, polysorbate 20 (PS20) and polysorbate 80 (PS80) are the most commonly used ones.

Polysorbates possess a hydrophilic sorbitan "head group" where each of the four hydroxyl groups is bound to a polyoxyethylene (POE) polymer chain. One or more hydroxyl groups at the end of the POE polymer chain are esterified with fatty acids, the latter represent the hydrophobic moiety of the molecule. There can be many variations to this underlying structure arising from type (sorbitan and isosorbide) and stereochemistry of the head group, length of the POE chains, number of fatty acid esters (1 to 4) and fatty acid composition (degree of unsaturation of fatty acid, length of fatty acid).

However, the degradation of the polysorbate surfactant has been an issue of concern in the pharmaceutical industry over the past decade. The degradation of the polysorbate can lead to lowered surfactant protection of the active agent against interfacial stress. Unwanted degradation reactions can also lead to a wide range of degradation products, including free fatty acids, which form (sub)visible particles and can precipitate. These polysorbate degradation products have also an unwanted impact on the stability of the drug, in particular protein drugs such as antibodies, thereby reducing shelf life of the drug formulation. Protein aggregation and the subsequent particle formation is one of the most common manifestations of protein instability. It is therefore crucial to maintain intact polysorbates in drug products.

In terms of their degradation pathways, polysorbates are prone to hydrolysis and/or oxidation. According to previous studies, chemically induced hydrolysis of polysorbates is exacerbated when pharmaceutical compositions are stored at higher temperatures, e.g., during suboptimal or accelerated storage conditions, while auto-oxidation can be problematic even at relatively low temperatures (e.g., 5-25 °C) and under mildly acidic conditions (e.g. pH 5.5). The rates of these hydrolysis and auto-oxidation reactions have been shown to increase with increased temperature.

For the pharmaceutical industry, the characterization of polysorbates in pharmaceutical drug products is highly relevant due to their tendency to form degradation products, and therefore, the polysorbate content is commonly monitored at different stages of the drug product development.

Aside from polysorbates, the most commonly used excipients in pharmaceutical compositions, in particular biopharmaceutical compositions for parenteral administration, are buffers, which contribute significantly to the overall stability of a drug product through various mechanisms. Also, parenteral compositions should preferably be at physiological pH, especially for subcutaneous application, to minimise patient pain and local inflammation.

The preferred buffers in pharmaceutical compositions of this type (i.e., comprising protein drugs) comprise are histidine (His)-, citrate-, succinate-, acetate- and phosphate buffers.

Sodium phosphate is one of the most commonly used buffers in parenteral formulations, as it mimics certain components of extracellular fluids, its pH is practically independent of temperature, and it is non-toxic to cells. However, sodium phosphate has the potential to precipitate during the freezing stage of lyophilization, which can cause local changes in pH. Thus, sodium phosphate should be avoided, especially during the processing of bulk drug substances or during lyophilization, as changes in the pH of the environment have the potential to destabilize protein drugs.

Citrate buffer is used in over 100 approved injectable drug products. It has anti-oxidant effects, a long history of use, and a proven safety profile. However, on the downside, citrate buffer solutions can induce pain upon parenteral (subcutaneous) administration. The use of acetic acid buffer is advisable for protein formulations that are stable in solution under acidic conditions, but should be avoided during lyophilisation due to rapid sublimation and consequent pH changes that may detrimentally affect the stability of the active agent.

Histidine buffers have become increasingly popular especially for biopharmaceutical formulations in recent years, and they are ideal for use under neutral pH conditions in both, liquid and lyophilised states. Their common use is a consequence of their unique buffering activity, painless parenteral application, antioxidant characteristics, and compatibility with the protein drugs and other formulation excipients. Histidine buffers also improve the physical properties of drug products, such as reducing the solution viscosity, which is desirable during the manufacturing process of the dosage forms and for therapeutic proteins, such as monoclonal antibodies (mAbs) at ultra-high concentrations in solution.

However, it has been reported that a buffer system of histidine and histidine acetate promoted oxidative degradation. In line with these findings, the present inventors found that a buffer system of histidine and histidine chloride also led to a considerable degradation of polysorbates in pharmaceutical compositions.

Despite the common use of polysorbates and buffers in pharmaceutical compositions, particularly in biopharmaceutical drug products, only limited data are available on the influence of buffers on the chemical stability of polysorbates.

For Example, WO 2019/106206 A1 reports stable, low viscosity, high concentration liquid pharmaceutical compositions of an anti-IL-12/23p40 antibody comprising defined concentration ranges of the antibody, a sugar, a non-ionic surfactant, and a histidine buffer with or without an inorganic salt particularly suitable for subcutaneous administration and for use in treating immune-mediated diseases in which IL-12 and/or IL-23 play a role. EP 3 156 071 A1 discloses stable and convenient aqueous adalimumab formulations by incorporating a histidine buffer solution and a phosphate buffer solution in combination. Meanwhile, EP 3 607 969 A1 teaches a pharmaceutical composition, which comprises infliximab, a histidine buffer system, a structure protectant and a surfactant, wherein the concentration of histidine in the histidine buffer system is approximately 1-200 mM. Lastly, WO 2018/060210 A1 discloses a liquid pharmaceutical composition in the pH range of 5.5 to 7.5 and comprising an interleukin-6 receptor, a histidine buffer, a polyol (such as a sugar alcohol), a free amino acid, a surfactant, and optionally a salt.

Hence, there is a need for buffer systems that combine the advantages of histidine as a buffering agent for pharmaceutical drug products, and that avoid polysorbate degradation during the formulation of the active agent, as well as in the final drug product, e.g., during storage.

### Figures

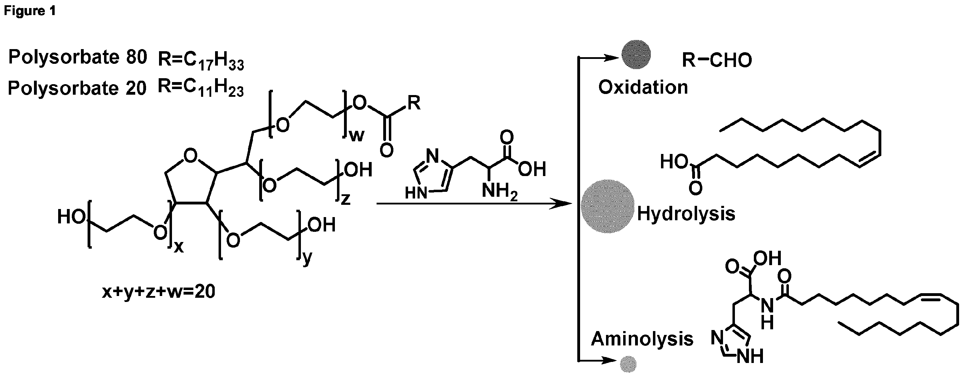

**Figure 1****:** Degradation of polysorbates in pharmaceutical compositions comprising histidine chloride buffer salt takes place via three mechanistic pathways, wherein hydrolysis is the predominant pathway. Oxidation, hydrolysis and aminolysis each result in different degradation products, which in some cases are poorly soluble in water and therefore prone to aggregation, and thus may result in the formation of precipitates. As a result of polysorbate degradation, the concentration and hence the stabilizing activity of polysorbate is reduced.

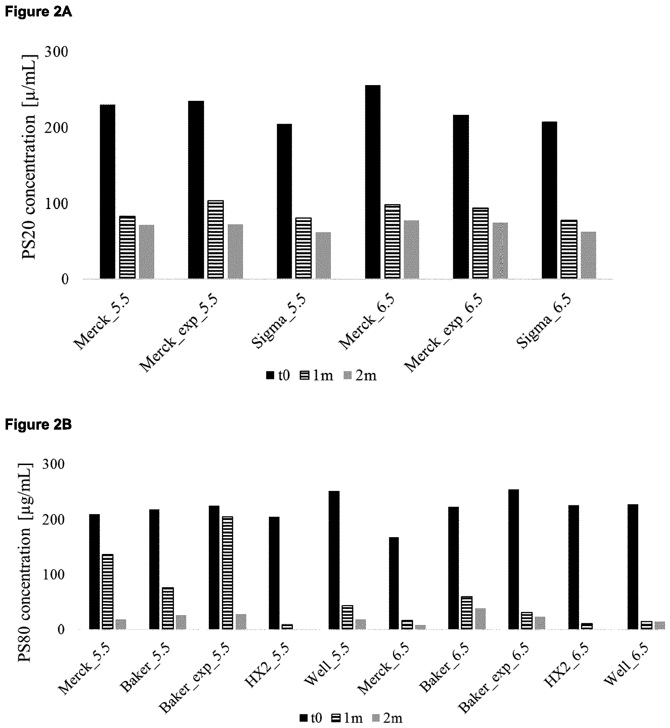

**Figure 2****:** Degradation of PS20 (Figure 2A) and PS80 (Figure 2B) in histidine chloride buffer solution. PS20 and PS80 were obtained from different manufacturers (see table 1) and subjected to accelerated stability testing at pH 5.5 and pH 6.5 ("exp" = expired). Samples were taken at the start of the experiment (t0), after one month (1m) and after two months (2m).

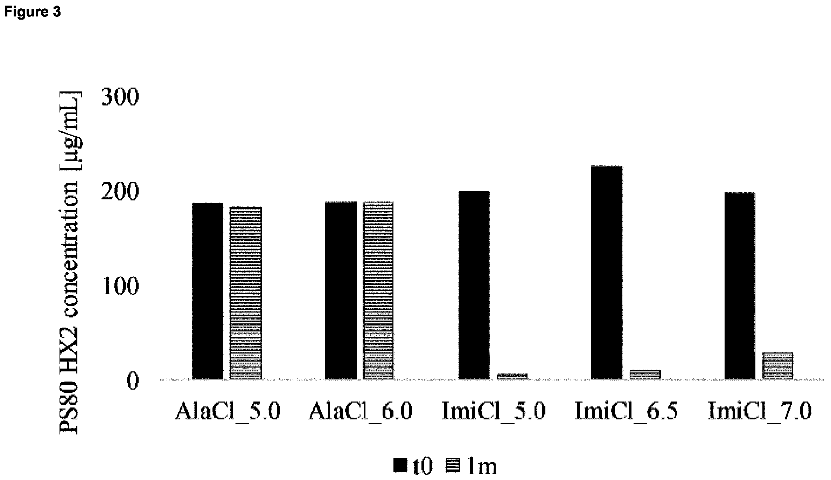

**Figure 3****:** Stability of PS80 HX2 in solutions containing alanine chloride buffer salt (AlaCI) at pH 5.0 or pH 6.0 or imidazole chloride buffer salt (ImiCI) at pH 5.0, pH 6.5 or pH 7.0. The assay was performed under accelerated storage conditions. Samples taken after 1 month (1m) showed no degradation for the samples containing alanine chloride buffer salt, whereas PS80 HX2 was degraded almost entirely in the samples containing imidazole chloride buffer salt across the tested pH range.

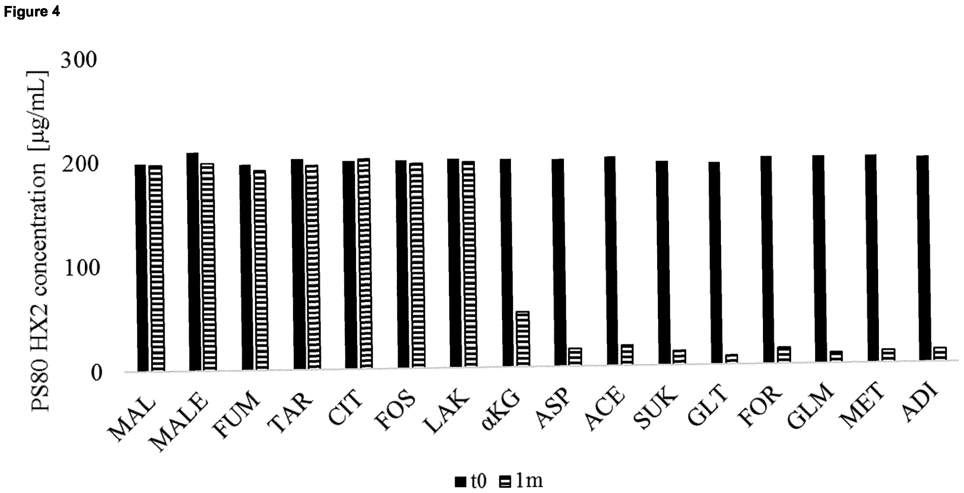

**Figure 4****:** Stability of PS80 in solutions comprising histidine buffer salts with various counter ions. The following histidine buffer salts were used: histidine malate (MAL), histidine maleate (MALE), histidine fumarate (FUM), histidine tartrate (TAR), histidine citrate (CIT), histidine phosphate (FOS), histidine lactate (LAK), histidine acetate (ACE), histidine glutarate (GLT), histidine succinate (SUK), histidine adipate (ADI), histidine α-ketoglutarate (αKG), histidine glutamate (GLM), histidine aspartate (ASP), histidine formiate (FOR) and histidine mesylate (MET). The concentration of PS80 HX2 is shown at each time point for all of the tested buffer salts.

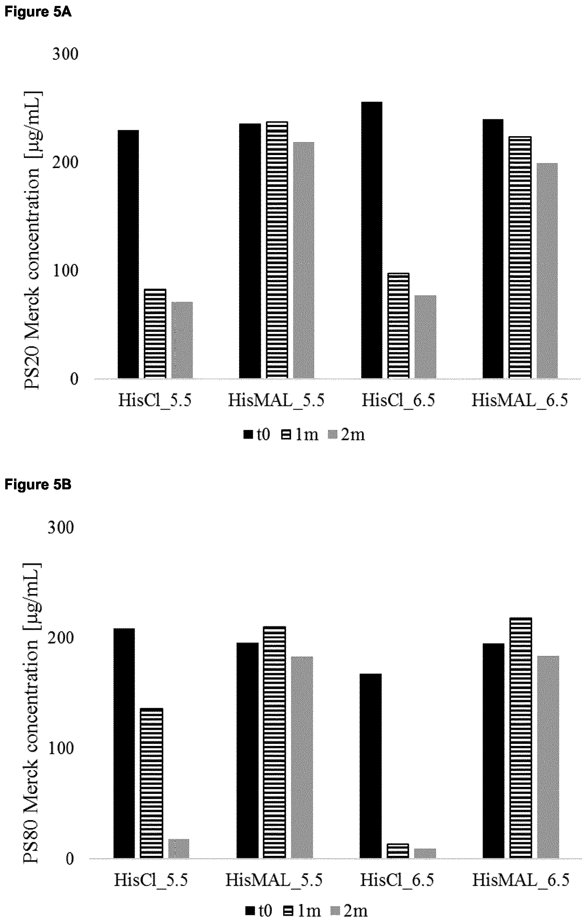

**Figure 5****:** Stability of PS20 (Figure 5A) and PS80 (Figure 5B) in solutions comprising histidine chloride or histidine malate buffer salts. Stability of PS20 (Merck) or PS80 (Merck) under accelerated stability testing conditions in the presence of histidine chloride (HisCI) or histidine malate (HisMAL) at pH 5.5 or pH 6.5.

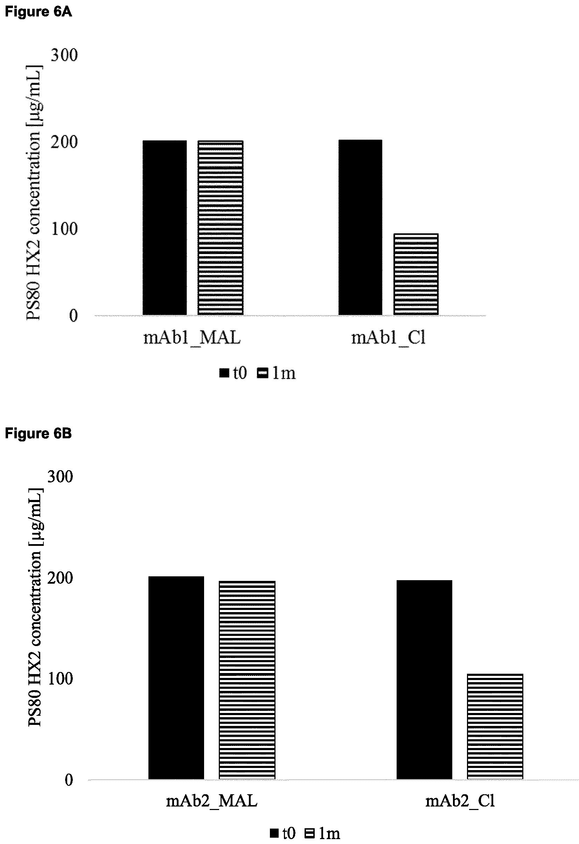

**Figure 6****:** Stability of PS80 in solutions comprising a histidine buffer salt and an active agent. Stability of PS80 HX2 (see table 1) in the presence of histidine chloride (CI) or histidine malate (MAL) buffer salts. The solutions further comprised one of two exemplary monoclonal antibodies as model substances for biopharmaceutical active agents. In the presence of either mAb1 (Figure 6A) or mAb2 (Figure 6B) significant degradation of PS80 was observed for the solutions comprising histidine chloride buffer salt, while no significant degradation was observed for the solutions comprising histidine malate buffer salt.

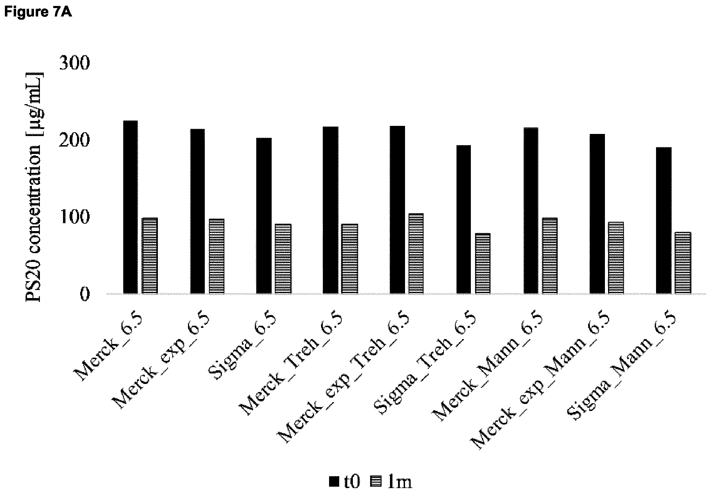

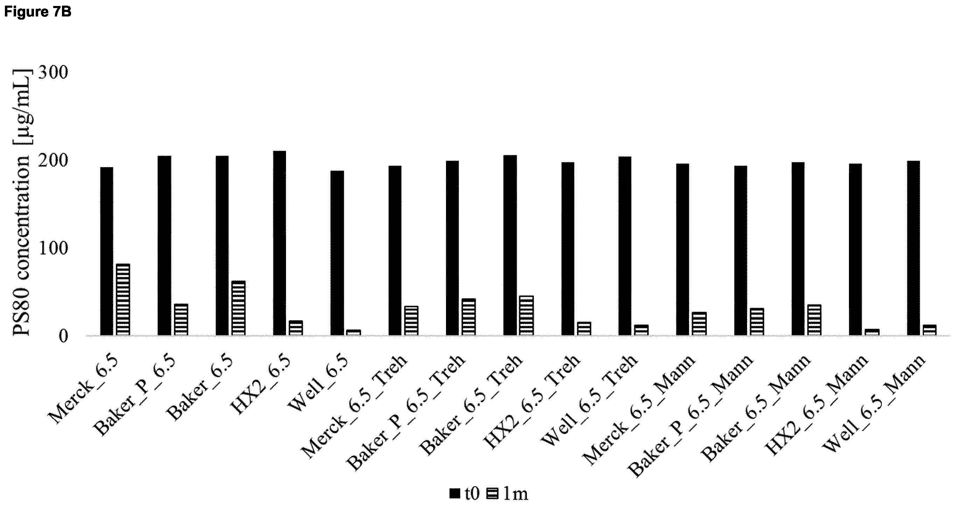

**Figure 7****:** Stability testing of PS20 (Fig. 7A) and PS80 (Fig. 7B) in the presence of the additional excipients mannitol (Mann) and trehalose (Treh) in HisCl buffer: PS20 and PS80 from various manufacturers were submitted to accelerated stability testing in the presence of histidine chloride buffer salt. Some of the samples additionally contained trehalose and mannitol as exemplary excipients of pharmaceutical compositions.

### Summary of the Invention

In the first aspect, the invention relates to a stabilized pharmaceutical composition comprising an active agent, polysorbate, and a histidine buffer consisting of histidine and a histidine buffer salt, wherein the histidine buffer salt is selected from the group consisting of histidine malate, histidine maleate, histidine fumarate, histidine tartrate, histidine citrate, histidine lactate, and mixtures thereof; and wherein the buffering salt is not histidine chloride or histidine acetate.

In one embodiment, the polysorbate is selected from the group consisting of polysorbate 20, polysorbate 21, polysorbate 40, polysorbate 60, polysorbate 61, polysorbate 65, polysorbate 80, polysorbate 81, polysorbate 85, polysorbate 120, and mixtures thereof.

In a further embodiment, the stabilized pharmaceutical composition comprises the polysorbate in a concentration of from about 0.0001% (w/v) to about 1.0% (w/v), preferably in a concentration of from about 0.01% (w/v) to about 0.25% (w/v).

In yet another embodiment, the stabilized pharmaceutical composition comprises the the histidine buffer salt in a concentration of from about 1 mM to about 200 mM, preferably in a concentration of from about 5 mM to about 100 mM, more preferably from about 10 mM to about 50 mM, most preferably in a concentration of about 20 mM.

In a further embodiment, the pH of the composition is from about pH 4.0 to about pH 8.5, preferably from about pH 5.5 to about pH 7.0.

In a further embodiment, the composition comprises a therapeutically effective amount of the active agent.

In an even further embodiment, the active agent is a protein, preferably a monoclonal or a polyclonal antibody, or an antigen-binding fragment thereof.

In a second aspect, the invention related to a method for preparing a stabilized pharmaceutical composition, which comprises including a histidine buffer consisting of histidine and a histidine buffer salt into a pharmaceutical composition comprising an active agent and polysorbate, wherein the histidine buffer salt is selected from the group consisting of histidine malate, histidine maleate, histidine fumarate, histidine tartrate, histidine citrate, histidine lactate, and mixtures thereof; and wherein the buffering salt is not histidine chloride or histidine acetate.

Lastly, in the final aspect of the invention, the invention relates to the use of a histidine buffer salt selected from the group consisting of histidine malate, histidine maleate, histidine fumarate, histidine tartrate, histidine citrate, histidine lactate, and mixtures thereof, for stabilizing polysorbate in a pharmaceutical composition comprising a histidine buffer consisting of histidine and a histidine buffer salt, preferably in a liquid pharmaceutical composition; wherein the buffering salt is not histidine chloride or histidine acetate.

### Detailed Description of the Invention

As used herein, the general embodiments "comprising" or "comprised" encompass the more specific embodiment "consisting of". Furthermore, singular and plural forms are not used in a limiting way. According to this disclosure, the singular forms "a", "an" and "the" designate both the singular and the plural, unless expressly stated to designate the singular only.

The present invention provides a stabilized pharmaceutical composition comprising an active agent, polysorbate, and a histidine buffer consisting of histidine and a histidine buffer salt, wherein the histidine buffer salt is selected from the group consisting of histidine malate, histidine maleate, histidine fumarate, histidine tartrate, histidine citrate, and histidine lactate, and mixtures thereof; and wherein the buffering salt is not histidine chloride or histidine acetate.

The terms "buffer", "buffering solution" or "buffering system" as used herein refer to a solution of a predefined pH, which is essentially maintained when (minor) amounts of acid or bases are added. Thus, a buffer is used to maintain the pH of a composition at or near a predetermined value. Typically, a buffer system consists of a weak base and a conjugated dissociated salt thereof, or a weak acid and a conjugated dissociated salt thereof. For example, an acetate buffer is a mixture of acetic acid (i.e., the weak acid) and sodium acetate (i.e., the conjugated dissociated salt thereof). The function of a buffering system is to prevent a rapid change in pH when a buffered composition is subjected to, e.g., rapid temperature changes, lyophilization, long term storage or the addition of acidic or basic compounds. A buffer acts by donating or accepting protons in the pH range around its pKₐ (acid dissociation constant). In general, a buffer exerts maximum buffering capacity at pH = pKₐ, and its ability to maintain pH declines as the pH moves away from the pKₐ of the buffer. The choice of the appropriate buffer system generally depends on the pH required.

A histidine buffer consists of histidine (i.e., a weak base) and an acid addition salt thereof. For example, a "histidine/histidine acetate buffer" (also referred to as "histidine acetate buffer" (HisACE)) consists of histidine and histidine acetate (i.e., the conjugated dissociated salt of histidine). For the purposes of the present invention, the latter species is referred to as the "histidine buffer salt".

The term "histidine buffer salt" (or "His buffer salt"), as used herein thus refers to a salt comprising 2-amino-3-(1H-imidazol-4-yl)propanoic acid (histidine) in the form of a cationic species and an anionic counter ion.

In the histidine buffer of the invention, the histidine as well as the histidine buffer salt may comprise histidine in the form of L-histidine or D-histidine, or a mixture thereof. Preferably, histidine and the histidine buffer salt according to the invention comprise histidine in the form of L-histidine. Hydrates of histidine as well as of the histidine buffer salts are included by the terms "histidine" or "histidine buffer salt", respectively.

The histidine buffer salt comprised in the stabilized pharmaceutical composition according to the invention is selected from the group consisting of histidine malate, histidine maleate, histidine fumarate, histidine tartrate, histidine citrate, histidine lactate, and mixtures thereof. The buffering salt comprised in the stabilized pharmaceutical composition is not histidine chloride or histidine acetate. Hence, the anionic counter ion of histidine is malate, maleate, fumarate, tartrate, citrate, lactate, and mixtures thereof.

In one embodiment, the histidine buffer salt is selected from the group consisting of histidine malate, histidine maleate, histidine fumarate, histidine tartrate, histidine citrate, histidine lactate, and mixtures thereof. In another embodiment, the histidine buffer salt is selected from the group consisting of histidine malate, histidine maleate, histidine fumarate, histidine tartrate, histidine lactate, and mixtures thereof. In yet another embodiment, the histidine buffer salt is selected from the group consisting of histidine malate, histidine maleate, histidine fumarate, histidine tartrate, histidine lactate, and mixtures thereof.

It has been surprisingly found that the pharmaceutical compositions of the invention that use said histidine buffer comprising a buffering salt with the above specific counter ions avoid polysorbate degradation, for example, during the production of the active agent, the subsequent formulation process, and in particular in the final composition, e.g., during storage or use.

The stabilized pharmaceutical composition of the invention may comprise any suitable polysorbate known to the skilled artisan for that purpose. "Polysorbate", sometimes also referred to as "Tween^{®}", refers to a class of amphiphilic, non-ionic surfactants that are derived from ethoxylated sorbitan (a derivative of sorbitol), esterified with fatty acids. Polysorbates are commonly manufactured by ethoxylation of sorbitan, prior to esterification with a fatty acid. The ethoxylation process leaves the sorbitan core with a defined number of repeat units of polyethylene glycol that are distributed across four different attachment sites (hydroxyl groups) of the sorbitan molecule. Accordingly, the term "polysorbate" refers to a mixture composed of chemical species that vary *inter alia* according to the specific distribution of polyethylene glycol units across the sorbitan hydroxyl groups.

Polysorbates are commonly named according to the following nomenclature. The first digit indicates the primarily esterified fatty acid (2 = lauric acid, 4 = palmitic acid, 6 = stearic acid, 8 = oleic acid, 12 = isostearic acid). The second digit indicates the type of esterification (0 = a monoester comprising 20 polyoxyethylene units, 1 = a monoester comprising 4 or 5 polyoxyethylene units, 5 = a triester comprising 20 polyoxyethylene units). The term "PS20" or "polysorbate 20" refers to polyoxyethylene (20) sorbitan monolaurate. "PS80" or "polysorbate 80" refers to polyoxyethylene (20) sorbitan monooleate.

Thus, in the stabilized pharmaceutical composition of the invention, the polysorbate is selected from the group consisting of polysorbate 20, polysorbate 21, polysorbate 40, polysorbate 60, polysorbate 61, polysorbate 65, polysorbate 80, polysorbate 81, polysorbate 85, polysorbate 120, and mixtures thereof. In a preferred embodiment, the polysorbate is selected from the group consisting of polysorbate 20, polysorbate 60, polysorbate 80, and mixtures thereof. More preferably, the polysorbate is selected from the group consisting of polysorbate 20, polysorbate 80, and mixtures thereof. In a specific embodiment, the polysorbate is polysorbate 80. In another specific embodiment, the polysorbate is polysorbate 20.

The amount of polysorbate in the stabilized pharmaceutical composition of the invention may vary, and an optimal amount may be readily determined by a person skilled in the art. For example, the pharmaceutical composition may comprise polysorbate in a concentration of from about 0.0001% (w/v) to about 1.0% (w/v), or from about 0.001% (w/v) to about 0.5% (w/v), or from about 0.01% (w/v) to about 0.25% (w/v), e.g., in a concentration of about 0.02% (w/v).

Thus, the stabilized pharmaceutical composition according to the invention preferably comprises a polysorbate selected from the group consisting of polysorbate 20, polysorbate 21, polysorbate 40, polysorbate 60, polysorbate 61, polysorbate 65, polysorbate 80, polysorbate 81, polysorbate 85, polysorbate 120, and mixtures thereof in a concentration of from about 0.0001% (w/v) to about 1.0% (w/v).

More preferably, the pharmaceutical composition comprises a polysorbate selected from the group consisting of polysorbate 20, polysorbate 60, polysorbate 80, and mixtures thereof in a concentration of from about 0.001% (w/v) to about 0.5% (w/v).

Even more preferably, a polysorbate selected from the group consisting of polysorbate 20, polysorbate 80, and mixtures thereof in a concentration of from about 0.01% (w/v) to about 0.25% (w/v), and most preferably polysorbate 80 in a concentration of from about 0.01% (w/v) to about 0.25% (w/v).

The stabilized pharmaceutical composition according to the invention comprises the histidine buffer salt in a concentration suitable for pharmaceutical use. Suitable amounts may be readily determined by the skilled artisan. In specific embodiments, the pharmaceutical composition comprises the histidine buffer salt in a concentration of from about 1 mM to about 200 mM, or in a concentration of from about 5 mM to about 100 mM, or in a concentration of from about 10 mM to about 50 mM, for example in a concentration of about 20 mM.

In one embodiment, the stabilized pharmaceutical composition according to the invention comprises a polysorbate selected from the group consisting of polysorbate 20, polysorbate 21, polysorbate 40, polysorbate 60, polysorbate 61, polysorbate 65, polysorbate 80, polysorbate 81, polysorbate 85, polysorbate 120, and mixtures thereof in a concentration of from about 0.0001% (w/v) to about 1.0% (w/v), and the histidine buffer salt in a concentration of from about 1 mM to about 200 mM.

Preferably, the pharmaceutical composition comprises a polysorbate selected from the group consisting of polysorbate 20, polysorbate 60, polysorbate 80, and mixtures thereof in a concentration of from about 0.001% (w/v) to about 0.5% (w/v), and the histidine buffer salt in a concentration of from about 5 mM to about 100 mM; even more preferably, the pharmaceutical composition comprises a polysorbate selected from the group consisting of polysorbate 20, polysorbate 80, and mixtures thereof in a concentration of from about 0.01% (w/v) to about 0.25% (w/v) and the histidine buffer salt in a concentration of from about 10 mM to about 50 mM; or polysorbate 80 in a concentration of about 0.02% (w/v) and the histidine buffer salt in a concentration of about 20 mM.

The pH of the stabilized pharmaceutical composition according to the invention may be any pH that is suitable for pharmaceutical compositions within the pH range obtainable with a histidine buffer. Generally, the pH is adjusted to ensure stability of the active agent in the composition, while being as close as possible to the physiological pH at the intended site of administration or release (blood, muscle, lung, digestive tract, etc.). The pH of a given system generally varies as a function of temperature. As defined herein, pH values are indicated for pharmaceutical compositions at room temperature, i.e., at a temperature of from about 20°C to about 25°C. Generally, pH values of pharmaceutical compositions range from about pH 4.0 to about pH 8.5.

In one embodiment, the pH of the pharmaceutical composition according to the invention is from about pH 4.5 to about pH 8.0, more preferably from about pH 5.0 to about pH 7.5, even more preferably from about pH 5.5 to about pH 7.0, and most preferably from about 5.5 to about 6.5.

In one embodiment, the stabilized pharmaceutical composition according to the invention therefore comprises a polysorbate selected from the group consisting of polysorbate 20, polysorbate 21, polysorbate 40, polysorbate 60, polysorbate 61, polysorbate 65, polysorbate 80, polysorbate 81, polysorbate 85, polysorbate 120, and mixtures thereof in a concentration of from about 0.0001% (w/v) to about 1.0% (w/v), and the histidine buffer salt in a concentration of from about 1 mM to about 200 mM, wherein the pH of the composition is from about pH 4.0 to about pH 8.5.

Preferably, the pharmaceutical composition comprises a polysorbate selected from the group consisting of polysorbate 20, polysorbate 60, polysorbate 80, and mixtures thereof in a concentration of from about 0.001% (w/v) to about 0.5% (w/v), and the histidine buffer salt in a concentration of from about 5 mM to about 100 mM, wherein the pH of the composition is from about pH 4.5 to about pH 8.0.

More preferably the pharmaceutical composition comprises a polysorbate selected from the group consisting of polysorbate 20, polysorbate 80, and mixtures thereof in a concentration of from about 0.01% (w/v) to about 0.25% (w/v), and the histidine buffer salt in a concentration of from about 10 mM to about 50 mM, wherein the pH of the composition is from about pH 5.0 to about pH 7.5; or the pharmaceutical composition comprises polysorbate 80 in a concentration of about 0.02% (w/v) and the histidine buffer salt in a concentration of about 20 mM, wherein the pH of the composition is from about pH 5.5 to about pH 7.0.

The active agent comprised in the pharmaceutical composition according to the invention may be any type of active agent. It is, however, preferred that the active agent is a protein drug.

The term "protein" is used interchangeably with "amino acid residue sequences", "polypeptide" or "peptide" and refers to polymers of amino acids of any length. These terms also include proteins and peptides that are post-translationally modified through reactions that include, but are not limited to, glycosylation, acetylation, phosphorylation or protein processing. Modifications and changes, for example fusions to other proteins, amino acid sequence substitutions, deletions or insertions, can be made in the structure of a polypeptide while the molecule maintains its biological functional activity. For example certain amino acid sequence substitutions can be made in a polypeptide or its underlying nucleic acid coding sequence and a protein can be obtained with the same properties.

The term "polypeptide" means a sequence with more than 50 amino acids and the term "peptide" means sequences with up to 50 amino acids in length. However, the terms are used herein interchangeably.

The protein may be any protein for preventive or therapeutic use. Proteins for preventive use include for example protein antigens for use in vaccines. Proteins for therapeutic use include in an exemplary manner recombinantly produced proteins, such as interferons, blood coagulation factors, immunosuppressive factors, cytokines, growth factors, protein C, erythropoietin, streptokinase, asparaginase, urate oxidase or papain, fusion proteins, such as TNF-receptor fusion proteins and antibodies, or antigen-binding fragments thereof.

The term "antibody" refers to a protein consisting of one or more polypeptides substantially encoded by immunoglobulin genes. The recognized immunoglobulin genes include the kappa, lambda, alpha, gamma, delta, epsilon and mu constant regions genes as well as the myriad immunoglobulin variable region genes. As used herein, the term "antibody" includes a polyclonal, monoclonal, bispecific, multi-specific, human, humanized, or chimeric antibody.

The term "antibody" is used herein in its broadest sense and encompasses monoclonal antibodies (including full length monoclonal antibodies), polyclonal antibodies, chimeric antibodies, humanized antibodies, human antibodies, multispecific antibodies (e.g. bispecific antibodies), single domain antibodies, and antigen-binding antibody fragments (such as Fv, Fab, Fab', F(ab)2 or other antigen-binding subsequences of antibodies). The term "antibody" also encompasses antibody conjugates and fusion antibodies. Bispecific antibodies include BITE^{®} (Bispecific T-cell Engager) and DART^{®} (Dual-Affinity Re-Targeting) antibodies. Single domain antibodies include camelids antibodies. Full length "antibodies" or "immunoglobulins" are generally heterotetrameric glycoproteins of about 150 kDa, composed of two identical light and two identical heavy chains. Each light chain is linked to a heavy chain by one covalent disulphide bond, while the number of disulphide linkages varies between the heavy chains of different immunoglobulin isotypes. Each heavy and light chain also has regularly spaced intrachain disulphide bridges. Each heavy chain has an amino terminal variable domain (VH) followed by three carboxy terminal constant domains (CH). Each light chain has a variable N-terminal domain (VL) and a single C-terminal constant domain (CL). Antibodies can be assigned into different classes, based on the amino acid sequence of the constant domain of their heavy chains. Antibodies expressed from the kappa, lambda, alpha, gamma, delta, epsilon and mu constant regions genes give rise to the five major classes of immunoglobulins: IgA, IgD, IgE, IgG, and IgM. The heavy chain constant domains that correspond to the different classes of antibodies are called α, δ, ε, γ, and m, respectively. The antibody classes may be further divided into subclasses (isotypes), e.g., IgG1, IgG2, IgG3, IgG4, IgA, and IgA2. The term "antibody" further refers to a type of antibody comprising a plurality of individual antibodies having the same specificity (variable domain) and having the same constant domains.

The term "polyclonal antibody" as used herein refers to a mixture of immunoglobulin molecules that each react against different specific determinants (epitopes), which are typically secreted by different B cell lineages within the body.

The term "monoclonal antibody" (mAb) as used herein refers to an antibody obtained from a population of substantially homogeneous antibodies based on their amino acid sequence. Monoclonal antibodies are highly specific, being directed against a single antigenic site. Furthermore, in contrast to conventional (polyclonal) antibody preparations, which typically include different antibodies directed against different determinants (epitopes), each mAb is directed against a single determinant on the antigen. In addition to their specificity, the mAbs are advantageous in that they can be and are usually synthesized by cell culture (hybridomas, recombinant cells or the like) uncontaminated by other immunoglobulins. The mAbs taught herein include chimeric, humanized and human antibodies.

In a preferred embodiment, the active agent is a monoclonal or a polyclonal antibody, or an antigen-binding fragment thereof. Most preferably, the active agent is a monoclonal antibody, or an antigen-binding fragment thereof. In one embodiment, said monoclonal antibody is selected from the group consisting of adalimumab, aducanumab, alacizumab, alemtuzumab, alirocumab, ascrinvacumab, atezolizumab, atinumab, bapineuzumab, belimumab, benralizumab, bevacizumab, bimekizumab, blinatumomab, blosozumab, bococizumab, brentuximab, burosumab, caplacizumab, capromab, cemiplimab, certolizumab, cetuximab, crenezumab, daclizumab, daratumumab, demcizumab, denosumab, dinutuximab, dupilumab, eculizumab, efalizumab, elotuzumab, emicizumab, enoticumab, eptinezumab, etaracizumab, etokimab, etrolizumab, evinacumab, evolocumab, fasinumab, fulranumab, gantenerumab, golimumab, ibritumomab, icrucumab, idarucizumab, inclacumab, infliximab, inotuzumab, ipilimumab, isatuximab, ixekizumab, margetuximab, mepolizumab, mirikizumab, natalizumab, necitumumab, nesvacumab, nivolumab, obinutuzumab, olaratumab, opicinumab, orticumab, ozanezumab, palivizumab, pamrevlumab, panitumumab, pembrolizumab, pertuzumab, ponezumab, ralpancizumab, ramucirumab, raxibacumab, refanezumab, relatlimab, rinucumab, risankizumab, rituximab, romosozumab, sarilumab, siltuximab, solanezumab, stamulumab, tadocizumab, tanezumab, tocilizumab, trastuzumab, tremelimumab, ustekinumab, vedolizumab, vesencumab, abciximab, and zolbetuximab.

Chemical stability of antibodies has been found to depend on pH wherein slightly acidic conditions are favorable in many cases.

The active agent comprised in the pharmaceutical composition according to the invention is typically comprised therein in a therapeutically effective amount. As used herein, the term "therapeutically effective amount" refers to the amount of an active agent that, when administered to a patient, prevents, reduces or ameliorates the occurrence, progression, severity and/or duration of a condition or disease. For example, the pharmaceutical composition according to the invention comprises the active agent in a concentration of from about 0.1 mg/mL to about 250 mg/mL, or from about 0.5 mg/mL to about 150 mg/mL, or from about 0.8 mg/mL to about 100 mg/mL, or from about 1 mg/mL to about 50 mg/mL.

Accordingly, in one embodiment, the stabilized pharmaceutical composition of the invention comprises a polysorbate selected from the group consisting of polysorbate 20, polysorbate 21, polysorbate 40, polysorbate 60, polysorbate 61, polysorbate 65, polysorbate 80, polysorbate 81, polysorbate 85, polysorbate 120, and mixtures thereof in a concentration of from about 0.0001% (w/v) to about 1.0% (w/v), the histidine buffer salt in a concentration of from about 1 mM to about 200 mM, and a therapeutically effective amount of an active agent which is a protein, preferably a monoclonal or a polyclonal antibody or an antigen-binding fragment thereof, more preferably a monoclonal antibody, or an antigen-binding fragment thereof.

Preferably, the stabilized pharmaceutical composition of the invention comprises a polysorbate selected from the group consisting of polysorbate 20, polysorbate 60, polysorbate 80, and mixtures thereof in a concentration of from about 0.001% (w/v) to about 0.5% (w/v), the histidine buffer salt in a concentration of from about 5 mM to about 100 mM, wherein the pH of the composition is from about pH 4.5 to about pH 8.0, and a therapeutically effective amount of an active agent which is a protein, preferably a monoclonal or a polyclonal antibody or an antigen-binding fragment thereof, more preferably a monoclonal antibody, or an antigen-binding fragment thereof.

More preferably, the stabilized pharmaceutical composition of the invention comprises a polysorbate selected from the group consisting of polysorbate 20, polysorbate 80, and mixtures thereof in a concentration of from about 0.01% (w/v) to about 0.25% (w/v), the histidine buffer salt in a concentration of from about 10 mM to about 50 mM, wherein the pH of the composition is from about pH 5.0 to about pH 7.5, and a therapeutically effective amount of an active agent which is a protein, preferably a monoclonal or a polyclonal antibody or an antigen-binding fragment thereof, more preferably a monoclonal antibody, or an antigen-binding fragment thereof.

In a specific embodiment, the stabilized pharmaceutical composition of the invention comprises polysorbate 80 in a concentration of about 0.02% (w/v), the histidine buffer salt in a concentration of about 20 mM, wherein the pH of the composition is from about pH 5.5 to about pH 7.0, and a therapeutically effective amount of an active agent which is a protein, preferably a monoclonal or a polyclonal antibody or an antigen-binding fragment thereof, more preferably a monoclonal antibody, or an antigen-binding fragment thereof.

The stabilized pharmaceutical composition of the invention may comprise further additives or excipients. Common excipients for pharmaceutical compositions are bulking agents, stabilizers, tonicity modifiers such as NaCl and mannitol, antioxidants such as L-methionine, chelating agents such as EDTA or further surfactants. Typical excipients for pharmaceutical compositions are described in Remington's Pharmaceutical Sciences 16th edition, Osol, A. Ed. (1980).

In a particularly preferred embodiment, the buffering salt comprised in the stabilized pharmaceutical composition according to the invention is not histidine glutarate, histidine succinate, histidine adipate, histidine α-ketoglutarate, histidine glutamate, histidine aspartate, histidine formiate and/or histidine mesylate.

Preferably, the pharmaceutical composition of the invention is a liquid composition or a lyophilized composition. In a more preferred embodiment, the pharmaceutical composition according to the invention is a liquid composition, most preferably a liquid aqueous composition.

The pharmaceutical composition according to the invention may be suitable for any type or route of administration. In preferred embodiments, the composition is for parenteral administration, such as for intravenous, subcutaneous or intramuscular administration, more preferably for intravenous or subcutaneous administration and most preferably for intravenous administration.

The pharmaceutical composition in accordance with the invention is generally in a microbiologically sterile form, and may conveniently be contained or stored in a sealed, sterile container such as a vial, syringe or capsule and stored at a desired temperature.

Chemical polysorbate degradation mediated by histidine buffer salts of the prior art such as histidine chloride takes place along three major mechanistic pathways, which include ester hydrolysis, oxidation, and ester aminolysis (Fig. 1), wherein ester hydrolysis is the predominant pathway. Ester hydrolysis results in the formation of free fatty acids that may precipitate as a result of poor solubility in the stabilized pharmaceutical composition. Oxidation in particular occurs at the ethylene oxide moiety of the polysorbate and the double bond of the fatty acid moiety and leads to the formation of aldehydes, ketones, peroxides and epoxides. Histidine-mediated ester aminolysis leads to the formation of fatty acid-histidine amide adducts. Aminolysis of, e.g., polysorbate 20, or polysorbate 80, results in the formation of lauroyl-histidine or oleoyl-histidine, respectively.

In the stabilized pharmaceutical composition of the invention that comprises a histidine buffer with the specific histidine buffer salt(s) as defined above, the polysorbate degradation is reduced relative to the same composition comprising histidine chloride as the histidine buffer salt. In particular, in the stabilized pharmaceutical compositions of the invention, the polysorbate ester hydrolysis is reduced relative to the same composition comprising histidine chloride as the histidine buffer salt. An exemplary, suitable standard test for determining and comparing polysorbate degradation is provided in the experimental section below (cf., under the heading *"Polysorbate content analysis").*

The invention further relates to a method for preparing a stabilized pharmaceutical composition, which comprises including a histidine buffer consisting of histidine and a histidine buffer salt into a pharmaceutical composition comprising an active agent and polysorbate, wherein the histidine buffer salt is selected from the group consisting of histidine malate, histidine maleate, histidine fumarate, histidine tartrate, histidine citrate, histidine lactate, and mixtures thereof; and wherein the histidine buffering salt is not histidine chloride or histidine acetate.

The method according to the invention provides a stabilized pharmaceutical composition wherein polysorbate degradation, e.g., by ester hydrolysis, is reduced compared to the same composition comprising histidine chloride as the histidine buffer salt.

In a further aspect, the invention relates to the use of a histidine buffer consisting of histidine and a histidine buffer salt selected from the group consisting of histidine malate, histidine maleate, histidine fumarate, histidine tartrate, histidine citrate, histidine lactate, and mixtures thereof, wherein the histidine buffering salt is not histidine chloride or histidine acetate, for stabilizing polysorbate in a pharmaceutical composition comprising polysorbate. Advantageously, said stabilizing leads to reduced histidine-mediated polysorbate ester hydrolysis compared to a same composition comprising histidine chloride as the histidine buffer salt. Said pharmaceutical composition further comprises an active agent as defined hereinabove.

In one embodiment, the present invention provides the use of a histidine buffer salt selected from the group consisting of histidine malate, histidine maleate, histidine fumarate, histidine tartrate, histidine citrate, histidine lactate, and mixtures thereof, wherein the histidine buffer salt is not histidine chloride or histidine acetate, for stabilizing polysorbate in a pharmaceutical composition comprising polysorbate. In another embodiment, the present invention provides the use of a histidine buffer salt selected from the group consisting of histidine malate, histidine maleate, histidine fumarate, histidine tartrate, histidine lactate, and mixtures thereof, wherein the histidine buffer salt is not histidine chloride or histidine acetate, for stabilizing polysorbate in a pharmaceutical composition comprising polysorbate. In yet another embodiment, the present invention provides the use of a histidine buffer salt selected from the group consisting of histidine malate, histidine maleate, histidine fumarate, histidine tartrate, histidine lactate, and mixtures thereof, wherein the histidine buffer salt is not histidine chloride or histidine acetate, for stabilizing polysorbate in a pharmaceutical composition comprising polysorbate.

In a preferred use according to the invention, the pharmaceutical composition is a liquid pharmaceutical composition, more preferably a liquid aqueous composition.

### Examples

### Sample preparation and accelerated stability testing

PS20 or PS80 obtained from different manufacturers (see Table 1) was dissolved in 20 mM aqueous buffer at pH 5.5 or 6.5 in a glass vial to a final concentration of 0.02% (w/v) polysorbate. The buffer salts used in the histidine buffers investigated were histidine chloride (HisCI) and various further histidine buffer salts (see below, all obtained from Sigma-Aldrich (now Merck KgaA)). As the extent of polysorbate degradation was the greatest in the presence of HisCI buffer salt, this buffer salt was used as the reference buffer salt.

Degradation mechanisms of the polysorbates were explored using 20 mM imidazole chloride and 20 mM alanine chloride buffer salts. Also, HisCI buffer salt comprising solutions were prepared with the additional excipients of mannitol or trehalose (both Sigma-Aldrich) to determine the influence of sugars and their derivatives on polysorbate degradation. Finally, two different mAbs were used as the active agent that each contained 10 mg/mL mAb, 0.02% PS80 HX2 and 20 mM HisCI buffer salt at pH 6.5. The therapeutic protein solutions were provided by Lek d.d. (Mengeš, Slovenia). In all cases, the starting point samples were collected immediately and stored at -80°C. The vials were then placed into a 50°C incubator for up to 2 months, with samples collected at 1 month and further samples in some cases at 2 months. All of these samples were stored at -80°C for subsequent analysis.

Several polysorbate manufacturers and polysorbate quality grades were compared in this accelerated stability testing (see table 1). Quality grades of commercially available polysorbate differ *inter alia* in the degree of homogeneity of the fatty acid component. The predominant fatty acid component generally dictates the type of polysorbate. "All oleate" type polysorbate offers a higher degree of homogeneity with regard to the fatty acid component (≥98.0% oleic acid) compared to the commonly used "multi-compendial" grade.

**Table 1: Details for the selected PS20 and PS80 samples from the different manufacturers.**

| Polysorbate | Manufacturer | Grade | Notes |
|---|---|---|---|
| PS20 | Sigma-Aldrich | Multi-compendial | / |
| PS20 | Merck | Multi-compendial | / |
| PS20 | Merck | Multi-compendial | Expired |
| PS80 | Merck | Multi-compendial | / |
| PS80 | J. T. Baker | Multi-compendial | Expired |
| PS80 | J. T. Baker | Multi-compendial | / |
| PS80 (HX2) | NOF Corporation | All-oleate | / |
| PS80 | Well Chemicals | All-oleate | / |

### Polysorbate content analysis

The polysorbate contents were analysed and quantified using a method based on mixed-mode chromatography, as mixed anion exchange and reversed phase, combined with a charged aerosol detector (CAD). The external standard approach was used for quantification of the surfactants. Chromatographic analysis was carried out using the method of Hewitt *et al.* (Hewitt, D. et al. J. Chromatogr. A 1218, 2138-2145 (2011).

Analytes were separated using a Waters Oasis MAX column (30 µm particle size, 2.1 mm x 20 mm), column temperature 30°C, flow rate 1 ml/min and a total run time of 10 min. The injection volume was 10 µL. Initial conditions were set at 90% solvent A (2 % formic acid in purified water) and 10% solvent B (2 % formic acid in isopropanol). Solvent B was increased to 20% in the first minute and held for 2.4 min. POE sorbitan esters were eluted using a step gradient of 20% to 100% B over 0.1 min, followed by an equilibration step of 10% B for 0.9 min.

Liquid chromatography-MS grade formic acid, water and isopropanol were purchased from Merck KgaA (Darmstadt, Germany).

### Example 1: Analysis of polysorbate stability in the presence of histidine chloride buffer salt.

The stability of PS 20 (Fig. 2A) and PS80 (Fig. 2B) was tested in the presence of histidine chloride buffer salt under accelerated conditions (see *Methods*) at pH 5.5 and pH 6.5. Polysorbate of various manufacturers (see table 1) was used in the study. Polysorbates were used at a concentration of 0.02 % w/v, histidine chloride at 20 mM. Samples were taken at the beginning of the study (t0), after one month (1m) and after 2 months (2m) and analyzed as described above.

Quality grade and shelf-life of the polysorbates did not have relevant effects on their degradation. A less heterogeneous raw polysorbate material ("all-oleate") did not contribute to minimising the problems of polysorbate instability. For PS80, in addition to the multi-compendial grade (from Merck and Baker), there is a new generation of "superior or all-oleate" grade PS80, with two tested here (HX2 from NOF Corporation, and Well from Well Chemicals). The new all-oleate grade of PS80 degraded even faster than the multi-compendial grade PS80 (Fig. 2B). These data show that the correct choice of histidine buffer system, in which polysorbates do not degrade, is more important for the stability of polysorbate than the quality grade of the polysorbate employed.

There were also differences in the rates of degradation between the PS20 (Fig. 2A) and PS80 (Fig. 2B) samples from the same manufacturer, with the PS80 samples generally degrading faster. This effect is most pronounced after 2 months of accelerated stability testing. However, both PS20 and PS80 showed severe degradation under the tested conditions in the presence of histidine chloride buffer salt.

### Example 2: Comparison of polysorbate degradation in the presence of alanine chloride and imidazole chloride buffer salts at different pH values.

To assess whether the degradation mechanism is catalysed by the imidazole moiety of histidine, buffered solutions with imidazole chloride and, separately, buffered solutions with alanine chloride were prepared. Polysorbate 80 HX2 (NOF Corporation, see table 1) in a concentration of 0.02% w/v was used for the study, which was performed under accelerated conditions, as described above. Alanine chloride and imidazole chloride buffer salts were used at a concentration of 20 mM. Samples were taken at the beginning of the study (t0) and after one month under accelerated conditions (1 m). The samples were analysed as described above. The analytically determined concentration of PS80 HX2 for each of the experiments is depicted in Fig. 3.

Polysorbate degradation was detected only in the presence of imidazole chloride buffer salt, indicating that the imidazole moiety of histidine causes polysorbate degradation. The experiments further show that the observed polysorbate degradation occurs over a pH range of at least from about pH 5.0 to about pH 7.0.

### Example 3: Stability of polysorbate 80 in solutions comprising histidine buffer salts with various different counter ions.

To determine the influence of the anionic counter ions of the histidine buffer salt, PS80 HX2 was subjected to accelerated stability testing under the conditions described above. PS80 HX2 (see table 1) was used in a concentration of 0.02% w/v together with a histidine buffer salt in a concentration of 20 mM. The pH was adjusted to pH = 6.5. The study was performed under accelerated conditions. Samples were taken at the beginning of the study (t0) and after one month of storage (1m).

Histidine malate (MAL), histidine maleate (MALE), histidine fumarate (FUM), histidine tartrate (TAR), histidine citrate (CIT), histidine phosphate (FOS), histidine lactate (LAK), histidine acetate (ACE), histidine glutarate (GLT), histidine succinate (SUK), histidine adipate (ADI), histidine α-ketoglutarate (αKG), histidine glutamate (GLM), histidine aspartate (ASP), histidine formiate (FOR) and histidine mesylate (MET) were used.

The solutions comprising histidine malate (MAL), histidine maleate (MALE), histidine fumarate (FUM), histidine tartrate (TAR), histidine citrate (CIT), histidine phosphate (FOS) or histidine lactate (LAK) showed no significant degradation of polysorbate 80 HX2, whereas major to almost complete degradation was observed for solutions comprising histidine acetate (ACE), histidine glutarate (GLT), histidine succinate (SUK), histidine adipate (ADI), histidine α-ketoglutarate (αKG), histidine glutamate (GLM), histidine aspartate (ASP), histidine formiate (FOR) or histidine mesylate (MET) (Fig. 4).

Surprisingly, the counter ion used in the histidine buffer salt had a significant impact on PS stability under the tested conditions. The tested counter ions fall into two groups - stabilizing and destabilizing counter ions or "histidine buffer salts".

### Example 4: Stability of PS20 and PS80 in solutions comprising histidine chloride or histidine malate.

The stability of PS20 (Merck) and PS80 (Merck) were tested at different pH values (pH 5.5 and 6.5) in the presence of an exemplary destabilizing buffer salt (histidine chloride, HisCI) or an exemplary stabilizing buffer salt (histidine malate, HisMAL). Polysorbates were used in a concentration of 0.02% w/v, histidine buffer salts at a concentration of 20 mM. Testing was performed under accelerated conditions as described above. Samples were taken at the beginning of the study (t0), after one month (1m) and after two months (2m).

At both tested pH values, PS20 (Fig. 5A) and PS80 (Fig. 5B) showed degradation in the presence of HisCl, but significantly less degradation in the presence of HisMAL.

### Example 5: Stability of PS80 in solutions comprising a histidine buffer salt and an active agent.

To assess whether the observed stabilizing or destabilizing effects of histidine buffer salts can also be observed in presence of an active agent, PS80 HX2 (0.02% w/v) was subjected to accelerated stability testing as described above in the presence of either histidine chloride (CI) or histidine malate (MAL) and one of two exemplary monoclonal antibodies (mAb1 (Fig. 6A) or mAb2 (Fig. 6B). Histidine buffer salts were used at a concentration of 20 mM, antibodies at a concentration of 10 mg/mL. The pH was adjusted to pH = 6.5. Samples were taken at the beginning of the study (t0) and after one month (1m) and analyzed as described above (Fig. 6).

Similar to the previous experiments, polysorbate 80 showed significantly faster degradation in the presence of histidine chloride buffer salt compared to histidine malate buffer salt.

### Example 6: Stability testing in the presence of the additional excipients mannitol (Mann), trehalose (Treh), and NaCl in histidine chloride based buffer.

Three common excipients of pharmaceutical compositions, mannitol (Mann), trehalose (Treh) and NaCl were tested for their influence on the stability of polysorbate. PS20 or PS80 (0.02 %w/v) from various manufacturers were submitted to accelerated stability testing in the presence of histidine chloride buffer salt in a concentration of 20 mM and at pH = 6.5. The samples additionally contained trehalose, mannitol or NaCl as exemplary excipients for pharmaceutical compositions. Samples were taken at the beginning of the experiment (t0) and after 1 month (1m) and analyzed as described above (Fig. 7).

The addition of trehalose, mannitol, NaCl and EDTA did not slow down the degradation of PS20 (Fig. 7A) or PS80 (Fig. 7B). The results obtained with NaCl are not shown.

## Claims

1. A stabilized pharmaceutical composition comprising an active agent, polysorbate, and a histidine buffer consisting of histidine and a histidine buffer salt, wherein the histidine buffer salt is selected from the group consisting of histidine malate, histidine maleate, histidine fumarate, histidine tartrate, histidine citrate, histidine lactate, and mixtures thereof; and wherein the buffering salt is not histidine chloride or histidine acetate.

2. The stabilized pharmaceutical composition of claim 1, wherein the polysorbate is selected from the group consisting of polysorbate 20, polysorbate 21, polysorbate 40, polysorbate 60, polysorbate 61, polysorbate 65, polysorbate 80, polysorbate 81, polysorbate 85, polysorbate 120, and mixtures thereof.

3. The stabilized pharmaceutical composition of claims 1 or 2, comprising the polysorbate in a concentration of from about 0.0001% (w/v) to about 1.0% (w/v), preferably in a concentration of from about 0.01% (w/v) to about 0.25% (w/v).

4. The stabilized pharmaceutical composition of any one of the preceding claims, comprising the histidine buffer salt in a concentration of from about 1 mM to about 200 mM, preferably in a concentration of from about 5 mM to about 100 mM, more preferably from about 10 mM to about 50 mM, most preferably in a concentration of about 20 mM.

5. The stabilized pharmaceutical composition of any one of the preceding claims, wherein the pH of the composition is from about pH 4.0 to about pH 8.5, preferably from about pH 5.5 to about pH 7.0.

6. The stabilized pharmaceutical composition of any one of the preceding claims, wherein the composition comprises a therapeutically effective amount of the active agent.

7. The stabilized pharmaceutical composition of any one of the preceding claims, wherein the active agent is a protein, preferably a monoclonal or a polyclonal antibody, or an antigen-binding fragment thereof.

8. The stabilized pharmaceutical composition of any one of the preceding claims, wherein the composition is a liquid composition or a lyophilized composition, preferably a liquid composition.

9. The stabilized pharmaceutical composition of any one of the preceding claims, for intravenous, subcutaneous or intramuscular administration, preferably for intravenous or subcutaneous administration.

10. The pharmaceutical composition of any one of the preceding claims, wherein the polysorbate degradation is reduced relative to the same composition comprising histidine chloride as the histidine buffer salt.

11. The pharmaceutical composition of claim 10, wherein the polysorbate ester hydrolysis is reduced relative to the same composition comprising histidine chloride as the histidine buffer salt.

12. A method for preparing a stabilized pharmaceutical composition, which comprises including a histidine buffer consisting of histidine and a histidine buffer salt into a pharmaceutical composition comprising an active agent and polysorbate, wherein the histidine buffer salt is selected from the group consisting of histidine malate, histidine maleate, histidine fumarate, histidine tartrate, histidine citrate, histidine lactate, and mixtures thereof; and wherein the buffering salt is not histidine chloride or histidine acetate.

13. Use of a histidine buffer salt selected from the group consisting of histidine malate, histidine maleate, histidine fumarate, histidine tartrate, histidine citrate, histidine lactate, and mixtures thereof, for stabilizing polysorbate in a pharmaceutical composition comprising a histidine buffer consisting of histidine and a histidine buffer salt, preferably in a liquid pharmaceutical composition; wherein the buffering salt is not histidine chloride or histidine acetate.

## Patentansprüche

1. Stabilisierte pharmazeutische Zusammensetzung, umfassend einen Wirkstoff, Polysorbat und einen Histidinpuffer, der aus Histidin und einem Histidinpuffersalz besteht, wobei das Histidinpuffersalz aus der Gruppe ausgewählt ist, die aus Histidinmalat, Histidinmaleat, Histidinfumarat, Histidintartrat, Histidincitrat, Histidinlactat und Mischungen davon besteht; und wobei das Puffersalz nicht Histidinchlorid oder Histidinacetat ist.

2. Stabilisierte pharmazeutische Zusammensetzung nach Anspruch 1, wobei das Polysorbat aus der Gruppe ausgewählt ist, die aus Polysorbat 20, Polysorbat 21, Polysorbat 40, Polysorbat 60, Polysorbat 61, Polysorbat 65, Polysorbat 80, Polysorbat 81, Polysorbat 85, Polysorbat 120 und Mischungen davon besteht.

3. Stabilisierte pharmazeutische Zusammensetzung nach Anspruch 1 oder 2, umfassend das Polysorbat in einer Konzentration von etwa 0,0001 % (w/v) bis etwa 1,0 % (w/v), vorzugsweise in einer Konzentration von etwa 0,01 % (w/v) bis etwa 0,25 % (w/v).

4. Stabilisierte pharmazeutische Zusammensetzung nach einem der vorhergehenden Ansprüche, umfassend das Histidinpuffersalz in einer Konzentration von etwa 1 mM bis etwa 200 mM, vorzugsweise in einer Konzentration von etwa 5 mM bis etwa 100 mM, stärker bevorzugt von etwa 10 mM bis etwa 50 mM, am stärksten bevorzugt in einer Konzentration von etwa 20 mM.

5. Stabilisierte pharmazeutische Zusammensetzung nach einem der vorhergehenden Ansprüche, wobei der pH der Zusammensetzung zwischen etwa pH 4,0 und etwa pH 8,5, vorzugsweise zwischen etwa pH 5,5 und etwa pH 7,0 liegt.

6. Stabilisierte pharmazeutische Zusammensetzung nach einem der vorhergehenden Ansprüche, wobei die Zusammensetzung eine therapeutisch wirksame Menge des Wirkstoffs umfasst.

7. Stabilisierte pharmazeutische Zusammensetzung nach einem der vorhergehenden Ansprüche, wobei der Wirkstoff ein Protein, vorzugsweise ein monoklonaler oder ein polyklonaler Antikörper, oder ein antigenbindendes Fragment davon ist.

8. Stabilisierte pharmazeutische Zusammensetzung nach einem der vorhergehenden Ansprüche, wobei die Zusammensetzung eine flüssige Zusammensetzung oder eine lyophilisierte Zusammensetzung, vorzugsweise eine flüssige Zusammensetzung, ist.

9. Stabilisierte pharmazeutische Zusammensetzung nach einem der vorhergehenden Ansprüche zur intravenösen, subkutanen oder intramuskulären Verabreichung, vorzugsweise zur intravenösen oder subkutanen Verabreichung.

10. Pharmazeutische Zusammensetzung nach einem der vorhergehenden Ansprüche, wobei die Polysorbatzersetzung reduziert ist, im Vergleich zu der gleichen Zusammensetzung, die Histidinchlorid als Histidinpuffersalz umfasst.

11. Pharmazeutische Zusammensetzung nach Anspruch 10, wobei die Polysorbatesterhydrolyse reduziert ist, im Vergleich zu der gleichen Zusammensetzung, die Histidinchlorid als Histidinpuffersalz umfasst.

12. Verfahren zur Herstellung einer stabilisierten pharmazeutischen Zusammensetzung, umfassend das Einbringen eines Histidinpuffers, der aus Histidin und einem Histidinpuffersalz besteht, in eine pharmazeutische Zusammensetzung, die einen Wirkstoff und Polysorbat umfasst, wobei das Histidinpuffersalz aus der Gruppe ausgewählt ist, die aus Histidinmalat, Histidinmaleat, Histidinfumarat, Histidintartrat, Histidincitrat, Histidinlactat und Mischungen davon besteht; und wobei das Puffersalz nicht Histidinchlorid oder Histidinacetat ist.

13. Verwendung eines Histidinpuffersalzes, ausgewählt aus der Gruppe, die aus Histidinmalat, Histidinmaleat, Histidinfumarat, Histidintartrat, Histidincitrat, Histidinlactat und Mischungen davon besteht, zur Stabilisierung von Polysorbat in einer pharmazeutischen Zusammensetzung, die einen Histidinpuffer umfasst, der aus Histidin und einem Histidinpuffersalz besteht, vorzugsweise in einer flüssigen pharmazeutischen Zusammensetzung; wobei das Puffersalz nicht Histidinchlorid oder Histidinacetat ist.

## Revendications

1. Composition pharmaceutique stabilisée comprenant un agent actif, le polysorbate et un tampon histidine constitué d'histidine et d'un sel de tampon histidine, le sel de tampon histidine étant choisi dans le groupe constitué par le malate d'histidine, le maléate d'histidine, le fumarate d'histidine, le tartrate d'histidine, le citrate d'histidine, le lactate d'histidine et leurs mélanges ; et le sel de tampon n'étant pas le chlorure d'histidine ou l'acétate d'histidine.

2. Composition pharmaceutique stabilisée selon la revendication 1, dans laquelle le polysorbate est choisi dans le groupe constitué par le polysorbate 20, le polysorbate 21, le polysorbate 40, le polysorbate 60, le polysorbate 61, le polysorbate 65, le polysorbate 80, le polysorbate 81, le polysorbate 85, le polysorbate 120 et leurs mélanges.

3. Composition pharmaceutique stabilise selon les revendications 1 ou 2, comprenant le polysorbate à une concentration d'environ 0,0001 % (p/v) à environ 1,0 % (p/v), de préférence à une concentration d'environ 0,01 % (p/v) à environ 0,25 % (p/v).

4. Composition pharmaceutique stabilisée selon l'une quelconque des revendications précédentes, comprenant le sel de tampon histidine à une concentration d'environ 1 mM à environ 200 mM, de préférence à une concentration d'environ 5 mM à environ 100 mM, davantage de préférence d'environ 10 mM à environ 50 mM, de préférence entre toutes à une concentration d'environ 20 mM.

5. Composition pharmaceutique stabilisée selon l'une quelconque des revendications précédentes, dans laquelle le pH de la composition est d'environ pH 4,0 à environ pH 8,5, de préférence d'environ pH 5,5 à environ pH 7,0.

6. Composition pharmaceutique stabilisée selon l'une quelconque des revendications précédentes, dans laquelle la composition comprend une quantité thérapeutiquement efficace de l'agent actif.

7. Composition pharmaceutique stabilisée selon l'une quelconque des revendications précédentes, dans laquelle l'agent actif est une protéine, de préférence un anticorps monoclonal ou polyclonal ou un de ses fragments de liaison à un antigène.

8. Composition pharmaceutique stabilisée selon l'une quelconque des revendications précédentes, dans laquelle la composition est une composition liquide ou une composition lyophilisée, de préférence une composition liquide.

9. Composition pharmaceutique stabilisée selon l'une quelconque des revendications précédentes, pour une administration intraveineuse, sous-cutanée ou intramusculaire, de préférence pour administration intraveineuse ou sous-cutanée.

10. Composition pharmaceutique selon l'une quelconque des revendications précédentes, dans laquelle la dégradation du polysorbate est réduite par rapport à la même composition comprenant du chlorure d'histidine comme sel de tampon histidine.

11. Composition pharmaceutique selon la revendication 10, dans laquelle l'hydrolyse de l'ester de polysorbate est réduite par rapport à la même composition comprenant du chlorure d'histidine comme sel de tampon histidine.

12. Procédé de préparation d'une composition pharmaceutique stabilisée, qui comprend l'inclusion d'un tampon histidine constitué d'histidine et d'un sel de tampon histidine dans une composition pharmaceutique comprenant un agent actif et le polysorbate, le sel de tampon histidine étant choisi dans le groupe constitué par le malate d'histidine, le maléate d'histidine, le fumarate d'histidine, le tartrate d'histidine, le citrate d'histidine, le lactate d'histidine et leurs mélanges ; et le sel de tampon n'étant pas le chlorure d'histidine ou l'acétate d'histidine.

13. Utilisation d'un sel de tampon histidine choisi dans le groupe constitué par le malate d'histidine, le maléate d'histidine, le fumarate d'histidine, le tartrate d'histidine, le citrate d'histidine, le lactate d'histidine et leurs mélanges, pour stabiliser le polysorbate dans une composition pharmaceutique comprenant un tampon histidine constitué d'histidine et d'un sel de tampon histidine, de préférence dans une composition pharmaceutique liquide ; le sel de tampon n'étant pas le chlorure d'histidine ou l'acétate d'histidine.
